# EUROPEAN PATENT APPLICATION

(11) **EP 4 706 610 A1**
(43) Date of publication of application: **11.03.2026**
(21) Application number: 25200664.8
(22) Date of filing: 05.09.2025
(51) Int. Cl.: A61F 13/01, A61F 13/15, A61F 13/551

(54) **HYGIENE PADS**

(30) Priority: 06.09.2024 GB 202413143; 25.02.2025 GB 202502739
(71) Applicant: The Cheeky Panda Limited, London EC3R 6EN (GB)
(72) Inventor: Chen, Jingyan, London, EC3R 6EN (GB)
(74) Representative: Murgitroyd & Company

(57) **Abstract**

There is provided a biodegradable hygiene pad comprising: an upper liner; an absorbent layer; a lower liner; and a back sheet, wherein the upper liner is arranged as a body side layer, wherein the absorbent layer is arranged between the upper liner and the lower liner, wherein the back sheet is arranged adjacent to the lower liner on a side opposite to the absorbent layer, wherein the back sheet is arranged as a garment side layer, and wherein the upper liner wherein the composition of the absorbent layer is at least 75% bamboo, and wherein the composition of the upper liner is at least 75% bamboo..

## Description

### Field of the Disclosure

The present invention relates to a hygiene pad, in particular a biodegradable hygiene pad.

### Background

Hygiene pads are designed to absorb bodily fluids and protect undergarments and clothing from staining. Absorbent hygiene pads are designed for use during menstruation, as well as for urinary and faecal incontinence. These products are designed such that when a pad has reached its fluid retention limit, it is disposed of by a user.

Hygiene pads typically comprise a layered arrangement of absorbent, fluid-retention materials and liquid-impermeable, leakproof materials. The absorbent layers of known hygiene pads are made from non-biodegradable materials, such as non-biodegradable plastics and synthetic polymers. The liquid-impermeable layers of known hygiene pads are also made from non-biodegradable materials, such as non-biodegradable plastics. Additionally, known hygiene pads contain non-biodegradable synthetic resins that are used as adhesives. Some pads are further treated with synthetic fragrance or chemicals.

As a result of the non-biodegradable core materials, known hygiene pads can take hundreds of years to decompose, contributing significantly to ocean pollution and landfill waste. Hygiene products containing substantial amounts of cotton have an added environmental impact, as cotton production requires excessive water consumption and generates pollution.

As such, there is a need to provide an improved hygiene pad.

### Summary

According to an aspect of the present invention, there is provided a biodegradable hygiene pad comprising: an upper liner; an absorbent layer; a lower liner; and a back sheet, wherein the upper liner is arranged as a body side layer, wherein the absorbent layer is arranged between the upper liner and the lower liner, wherein the back sheet is arranged adjacent to the lower liner on a side opposite to the absorbent layer, wherein the back sheet is arranged as a garment side layer, wherein the composition of the absorbent layer is at least 75% bamboo, and wherein the composition of the upper liner is at least 75% bamboo. As such, the hygiene pad would be made of sustainable materials, whilst ensuring reliability of fluid retention, hygiene and comfort of the user.

Unless otherwise specified, all percentages described herein refer to the weight of the respective material as a proportion of the total weight of the product. For example, when specifying that the absorbent layer is at least 75% bamboo, it is understood that the weight of the bamboo in the absorbent layer constitutes at least 75% of the total weight of the absorbent layer.

The hygiene pad of the present invention is a biodegradable hygiene pad. As such, the hygiene pad consists of biodegradable materials. As such, the hygiene pad is substantially absent of non-biodegradable materials.

Preferably, the composition of the absorbent layer is at least 80% bamboo. More preferably, the composition of the absorbent layer is at least 85% bamboo. More preferably, the composition of the absorbent layer is at least 90% bamboo. More preferably, the composition of the absorbent layer is at least 95% bamboo. More preferably, the composition of the absorbent layer is substantially 100% bamboo. As such, the absorbent layer comprises substantially a sustainable, biodegradable material.

Preferably, the composition of the upper liner is at least 80% bamboo. More preferably, the composition of the upper liner is at least 85% bamboo. More preferably, the composition of the upper liner is at least 90% bamboo. More preferably, the composition of the upper liner is at least 95% bamboo. More preferably, the composition of the upper liner is substantially 100% bamboo. As such, the upper liner comprises substantially a sustainable, biodegradable material.

Preferably, the lower liner comprises one or more materials selected from a group of biodegradable materials comprising: wood fibres, cotton fibres, synthetic polymers and any other biodegradable material. As such, the lower liner of the hygiene pad is biodegradable and comprises any number of biodegradable materials. Wood fibres provide a breathable, sustainable biodegradable material. Cotton fibres provide a natural, soft and breathable biodegradable material. Synthetic polymers provide a durable and soft biodegradable material. As such, the lower liner is substantially absent of non-biodegradable materials, breaks down naturally and does not contribute to ocean pollution and landfill waste.

Preferably, the biodegradable hygiene pad further comprises a lining paper arranged between the upper liner and the absorbent layer. As such, the lining paper protects the bamboo core and assists with absorption.

Preferably, the biodegradable hygiene pad further comprises glue. As such, the glue ensures the structural integrity of the hygiene pad, preventing the layers from shifting or separating during use and preventing fluid from leaking from the hygiene pad.

Preferably, the glue is arranged between the upper liner and the back sheet for sealing the upper liner to the back sheet. As such, the glue ensures that the absorbent layer remains contained within the pad.

Preferably, the glue is arranged between the lower liner and the back sheet for sealing the lower liner to the back sheet. As such, the glue ensures the structural integrity of the hygiene pad and prevents fluid from leaking between the lower liner and the back sheet.

Preferably, the bamboo of the upper liner is a bamboo fibre composition. Preferably the bamboo fibre composition of the upper liner comprises bamboo viscose. As such, the upper liner is breathable, soft, non-abrasive and comfortable.

Preferably the bamboo fibre composition of the upper liner comprises a hydrophilic surface layer. As such, the upper liner is designed for efficient absorption of liquids.

Preferably the bamboo fibre composition of the upper liner comprises non-woven bamboo fibres, preferably wherein the non-woven bamboo fibres are spun-lace bamboo fibres. As such, the bamboo fibres of the upper liner are soft, non-abrasive and comfortable.

Preferably, the lower liner comprises bamboo. Preferably the composition of the lower liner is at least 75% bamboo. More preferably, the composition of the lower liner is at least 80% bamboo. More preferably, the composition of the lower liner is at least 85% bamboo. More preferably, the composition of the lower liner is at least 90% bamboo. More preferably, the composition of the lower liner is at least 95% bamboo. More preferably, the composition of the lower liner is substantially 100% bamboo. As such, the lower liner comprises substantially a sustainable, biodegradable material.

Preferably the bamboo of the lower liner is a bamboo fibre composition. Preferably, the bamboo fibre composition of the lower liner comprises bamboo viscose. As such, the lower liner is breathable, soft, non-abrasive and comfortable.

Preferably the bamboo fibre composition of the lower liner comprises a hydrophilic surface layer. As such, the lower liner is designed for efficient handling of liquids.

Preferably the bamboo fibre composition of the lower liner comprises non-woven bamboo fibres, preferably wherein the non-woven bamboo fibres are spun-lace bamboo fibres. As such, the bamboo fibres of the lower liner are strong and durable, support the absorbent layer and add to the overall integrity of the pad.

Preferably the absorbent layer comprises a bamboo fibre composition. As such, the absorbent layer comprises a sustainable, biodegradable material.

Preferably the absorbent layer is substantially absent of cotton fibres. Preferably the absorbent layer is substantially absent of wood fibres. Preferably the absorbent layer is substantially absent of synthetic polymers. As such, the absorbent layer is substantially absent of non-biodegradable materials and does not contribute to ocean pollution and landfill waste. As such, unlike cotton production, the production of raw materials for the hygiene pad do not require excessive water consumption nor generate substantial pollution.

Preferably the absorbent layer bamboo fibre composition comprises individual bamboo fibres, wherein the fibres have a fibre length of preferably 0.5mm to 10mm, more preferably a length of 0.7mm to 7mm, most preferably a length of 1mm to 4mm. As such, the bamboo fibres are configured to provide a highly absorbent material.

Preferably, the upper liner has a material weight of preferably 0.01g to 1 g, more preferably a material weight of 0.2g to 0.7g, most preferably a material weight of 0.4g to 0.6g. Preferably, the absorbent layer has a material weight of preferably 1g to 8g, more preferably a material wight of 2g to 6g, most preferably a material weight of 3g to 5g. Preferably, the lower liner has a material weight of preferably 0.01g to 1 g, more preferably a material weight of 0.2g to 0.7g, most preferably a material weight of 0.4g to 0.6g.

Preferably the back sheet comprises a biodegradable plastic. As such, the back sheet breaks down naturally and does not contribute to ocean pollution and landfill waste.

Preferably the biodegradable plastic comprises one or more materials selected from a group of materials comprising: polylactic acid, polybutylene adipate terephthalate, polyhydroxyalkanoates, polybutylene succinate and starch. As such, the biodegradable plastic comprises biodegradable polymers and will break down into natural components.

Preferably the biodegradable plastic compromises polylactic acid and polybutylene adipate terephthalate, wherein preferably the polylactic acid comprises cornstarch, and wherein preferably the polybutylene adipate terephthalate comprises adipic acid, terephthalic acid and butanediol. As such the biodegradable plastic comprises renewable raw materials.

Preferably the hygiene pad has a thickness of 0.1mm to 5cm, more preferably a thickness of 0.1mm to 3mm, most preferably a thickness of 0.1mm to 1cm. As such, the hygiene pad is designed for the comfort of the user, whilst ensuring reliability of fluid retention.

Preferably the hygiene pad has an absorption rate of 0.5 seconds to 10 seconds, more preferably an absorption rate of 5 seconds to 9 seconds, most preferably an absorption rate of 7 seconds to 8 seconds, and wherein the hygiene pad has a water absorption ratio of preferably 5 to 100, more preferably a water absorption ratio of 7 to 20, most preferably a water absorption ratio of 9.5 to 12.1. As such, the hygiene pad has a high absorbency rate and liquid absorption ratio.

Preferably the hygiene pad further comprises a pair of tabs for securing the hygiene pad to a garment. As such, the tabs ensure that the hygiene pad does not bunch up or shift during use. Furthermore, the tabs ensure hygiene pad is kept in place to prevent leaking.

Preferably the hygiene pad further comprises an adhesive portion for securing the hygiene pad to a garment. As such, the pad is kept in place and prevented from shifting or moving. As such, the risk of leakage is reduced, especially during physical activities.

Preferably the hygiene pad is a biodegradable hygiene pad, and the hygiene pad is wrapped in a biodegradable wrapper made from cornstarch. As such, the hygiene pad and the hygiene pad wrapper are biodegradable and comprise sustainable materials.

According to another aspect of the invention, there is provided a biodegradable wrapper for wrapping the biodegradable hygiene pad, wherein the wrapper is made from cornstarch. As such, the wrapper is biodegradable and does not contribute to ocean pollution and landfill waste.

According to another aspect of the invention, there is provided a biodegradable sanitary article according to the description of the biodegradable hygiene pad. As such, the biodegradable sanitary pad would be made of sustainable materials, whilst ensuring reliability of fluid retention, hygiene and comfort of the user.

### Brief Description of the Drawings

Aspects of the present invention will now be described by way of example only and with reference to the accompanying drawings, in which:
Figure 1 depicts an expanded view of the layers of the hygiene pad in accordance with the present invention;
Figure 2 depicts an expanded view of the hygiene pad with a liner paper in accordance with an aspect of the present invention;
Figure 3 depicts an alternate expanded view of the hygiene pad in accordance with the present invention;
Figure 4 depicts an expanded view of the hygiene pad with glue in accordance with an aspect of the present invention;
Figure 5 depicts an expanded view of the hygiene pad with tabs in accordance with an aspect of the present invention;
Figure 6 depicts a view of the hygiene pad with tabs, sticker paper and a wrapper in accordance with an aspect of the present invention;
Figure 7 depicts a partially unfolded view of the hygiene pad with a wrapper and sticker paper in accordance with an aspect of the present invention; and
Figure 8 depicts an alternative configuration of the hygiene pad.

### Detailed Description

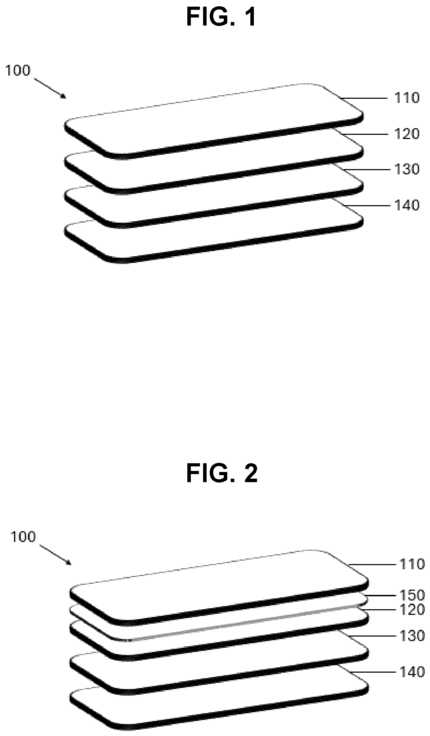

With reference to Figure 1, there is illustrated a biodegradable hygiene pad 100. The hygiene pad 100 comprises an upper liner 110, an absorbent layer 120, a lower liner 130, and a back sheet 140. When the hygiene pad 100 is in use, the upper liner 110 is in direct contact with the skin. The absorbent layer 120 is arranged between the upper liner 110 and the lower liner 130. The absorbent layer 120 is highly absorbent and designed to absorb and retain liquid. The lower liner 130 is configured to support the absorbent layer 120 and contributes to the overall structural integrity of the hygiene pad 100. The back sheet 140 is arranged adjacent to the lower liner 130 on a side opposite to the absorbent layer 120. The back sheet 140 is arranged as a garment side layer and provides a leak-proof barrier.

With reference to Figure 2, an example comprising a lining paper 150 is illustrated. The lining paper 150 is arranged between the upper liner 110 and the absorbent layer 120. The lining paper 150 is breathable and absorbent. As such, the lining paper is configured to protect the bamboo fibre inside the absorbent layer 120. The lining paper 150 is biodegradable and comprises biodegradable fibres. In the example illustrated in figure 2, the lining paper 150 comprises wood fibres. In an alternative example, the lining paper 150 comprises bamboo fibres.

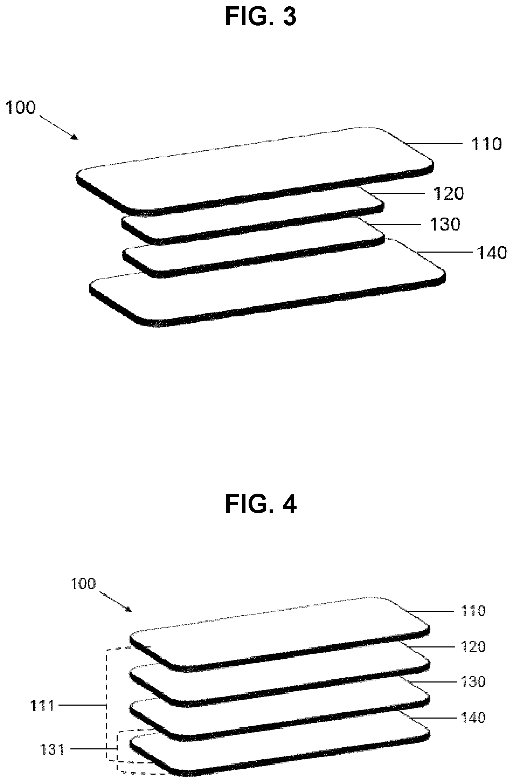

With reference to Figure 1, in an example, the upper liner 110, absorbent layer 120, lower liner 130, and back sheet 140 have the same surface area. With reference to Figure 3, in an alternative example, the upper liner 110, the absorbent layer 120, the lower liner 130, and the back sheet 140 comprise different surface areas. In the example illustrated in Figure 3, the back sheet 140 and the upper liner 110 have the same surface area, and the absorbent layer 120 and lower liner 130 have the same surface area. In the example illustrated in Figure 3, the surface area of the absorbent layer 120 and lower liner 130 is smaller than the surface area than the upper liner 110 and back sheet 140. In the example illustrated in Figure 1, the upper liner 110, absorbent layer 120, lower liner 130, and back sheet 140 have the same shape. In alternative aspects of the invention, the upper liner 110, absorbent layer 120, lower liner 130, and back sheet 140 have different shapes.

With reference to Figure 4, the hygiene pad 100 further comprises glue 111 and 131. The hygiene pad 100 is approximately 3% glue 111 and 131. The glue 111 and 131 comprises biodegradable materials and will break down naturally over time. When the biodegradable hygiene pad 100 is disposed, the glue 111 and 131 does not harm the environment. The glue 111 and 131 does not contribute to ocean pollution or landfill waste. In alternative examples, the glue is non-biodegradable. The glue 111 is arranged between the upper liner 110 and the back sheet 140 for sealing the upper liner 110 to the back sheet 140. The glue 131 is arranged between the lower liner 130 and the back sheet 140 for sealing the lower liner 130 to the back sheet 140. The arrangement of the glue ensures the structural integrity of the hygiene pad 100 and prevents leaking. In the example illustrated in Figure 4, the glue 111 is arranged around the outer perimeter of the upper liner 110 and the back sheet 140, and the glue 131 is arranged around the outer perimeter of the lower liner 130 and the back sheet 140. In alternative examples, the glue 111 and 131 may be arranged around the outer perimeter, or any other arrangement.

In the illustrated examples, the upper liner 110 and the lower liner 130 comprise a bamboo fibre composition. The bamboo fibre composition comprises bamboo viscose. The bamboo viscose has a fibre length of 20mm to 40mm. The bamboo fibre composition has a fibre diameter of 1µm to 30µm. The bamboo fibre composition comprises non-woven bamboo fibres. The non-woven bamboo fibres are spun-lace bamboo fibres.

The absorbent layer 120 may comprise substantially of bamboo. In other words, the absorbent layer 120 may be substantially free of materials other than bamboo. Numerically, the absorbent layer 120 may comprise a bamboo content of substantially 100%. Preferably, the absorbent layer 120 comprises a bamboo content of 100%. The absorbent layer 120 may be substantially absent of non-bamboo materials, such as cotton fibres, wood fibres and synthetic polymers.

Preferably, the absorbent layer 120 comprises a cotton fibre content of 0%, a wood fibre content of 0% and a synthetic polymer content of 0%. In the illustrated examples, the absorbent layer 120 is substantially 100% bamboo and is absent of any non-bamboo materials.

The absorbent layer 120 bamboo composition comprises individual bamboo fibres. In the illustrated examples, the absorbent layer 120 comprises individual bamboo fibres with an average length of 1.832mm.

The back sheet 140 comprises biodegradable plastic and will break down naturally over time. In the illustrated examples, the biodegradable plastic is made from polylactic acid made from cornstarch. In the illustrated examples, the biodegradable plastic comprises polybutylene adipate terephthalate made from adipic acid, terephthalic acid and butanediol. The biodegradable plastic comprises a combination of polylactic acid and polybutylene adipate terephthalate.

The thickness of the biodegradable hygiene pad 100 varies depending on the application of the hygiene pad 100. In an example, the hygiene pad 100 is a heavy flow hygiene pad and has a thickness of 5.5mm. In an alternative example, the hygiene pad 100 is a light flow hygiene pad and has a thickness of 5.16mm.

The absorption rate and the absorption ratio of the hygiene pad 100 may vary. In an example, the absorption rate is 7 second and the water absorption ratio is 9.5.

The density of the hygiene pad 100 may vary. In an example, the material weight of the upper liner is approximately 0.5g, the material weight of the absorbent layer is approximately 4g, and the material weight of the lower liner is approximately 0.5g.

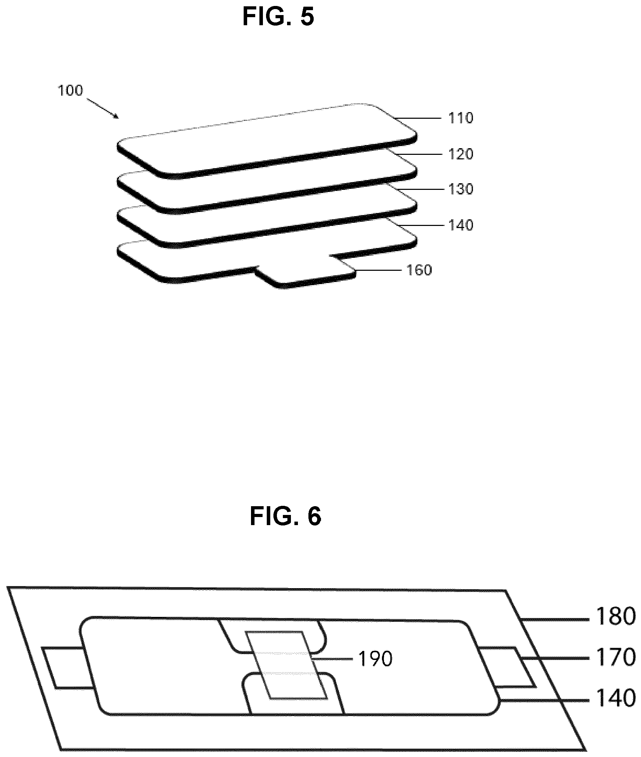

With reference to Figure 5 and Figure 6, the hygiene pad 100 comprises a pair of tabs 160, or wings, to secure the hygiene pad 100 to a garment. The garment may be an undergarment and the tabs 160 are designed to fold around the sides of the undergarment and help the hygiene pad 100 stay in place. In wrapping around the side of the undergarment, the tabs 160 also provide protection against side leaks. In the example illustrated in Figure 5, the tabs 160 comprise an upper liner 110 and a back sheet 140.

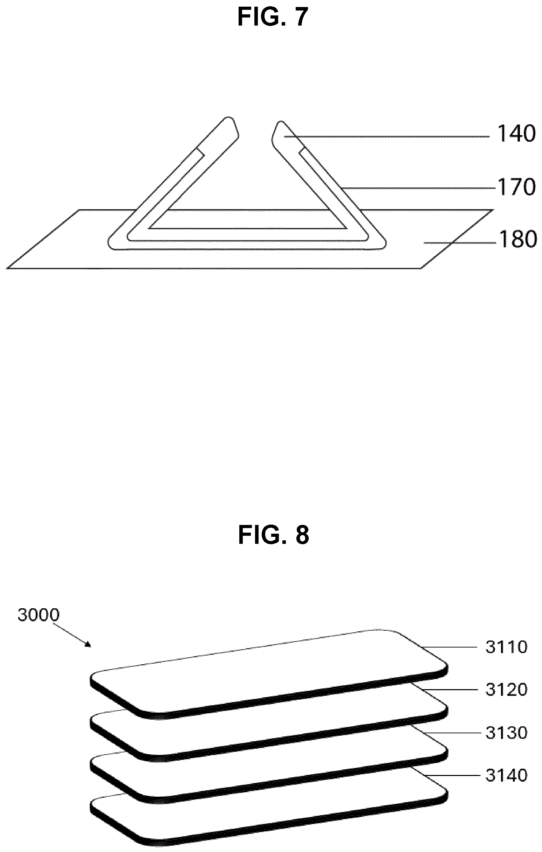

With reference to Figure 6 and Figure 7, the hygiene pad 100 comprises an adhesive portion on the garment side of the back sheet 140. The adhesive portion allows the hygiene pad 100 to stick to a garment. To prevent the adhesive portion from accidently sticking to materials when not in use, a sticker paper 170 is applied to the adhesive portion. The sticker paper 170 is biodegradable. In the example illustrated in Figure 6, the sticker paper 170 is longer than the length of the back sheet 140. In the example illustrated in Figure 7, the sticker paper 170 is shorter than the length of the back sheet 140.

In the example illustrated in Figure 6, the hygiene pad 100 comprises a tabs sticker paper 190. The tabs sticker paper 190 adheres to the tabs 160 and secures the hygiene pad 100 in a folded conformation. The tabs sticker paper 190 is biodegradable.

In use, the user would remove the sticker paper 170 from the hygiene pad 100 and expose the adhesive portion of the back sheet 140. In examples where the hygiene pad further comprises tabs 160 and a tabs sticker paper 190, the user would also remove the tabs sticker paper 190. The adhesive position would stick to the garment and the upper liner 110 would be positioned against the user's skin. When the hygiene pad 100 has reached its absorbance capacity, the hygiene pad 100 can be removed from the garment and the hygiene pad 100 is disposed of by the user.

With reference to Figure 6 and Figure 7, the hygiene pad 100 is packaged in a biodegradable wrapper 180 before use. The wrapper 180 protects the hygiene pad 100 and keeps it free of moisture and contaminants. The wrapper 180 is discreet and lightweight. The hygiene pad 100 is folded compactly inside the wrapper 180. The wrapper 180 is made from a biodegradable material. In the illustrated examples, the wrapper 180 comprises cornstarch.

With reference to Figure 6, the hygiene pad 100 is a biodegradable sanitary article. The biodegradable sanitary article may be a panty liner, a light flow pad, a heavy flow pad or a night pad. In the example illustrated in Figure 6, the hygiene pad 100 is a light flow pad.

With reference to Figure 8, there is illustrated an alternative configuration of a biodegradable hygiene pad 3000. The hygiene pad 3000 comprises an upper liner 3110, an absorbent layer 3120, a lower liner 3130, and a back sheet 3140. In the example illustrated in Figure 8, the absorbent layer 3120 is 75% bamboo and 25% wood fibres, and the upper liner 3110 is 75% bamboo and 25% wood fibres. The lower liner 3130 is substantially absent of bamboo. In the illustrated example, the lower liner comprises wood fibres and synthetic polymers.

With reference to Figure 8, when the hygiene pad 3000 is in use, the upper liner 3110 is in direct contact with the skin. The absorbent layer 3120 is arranged between the upper liner 3110 and the lower liner 3130. The absorbent layer 3120 is highly absorbent and designed to absorb and retain liquid. The lower liner 3130 is configured to support the absorbent layer 3120 and contributes to the overall structural integrity of the hygiene pad 3000. The back sheet 3140 is arranged adjacent to the lower liner 130 on a side opposite to the absorbent layer 3120. The back sheet 3140 is arranged as a garment side layer and provides a leak-proof barrier.

In an alternative embodiment of the example illustrated in Figure 8, the upper liner 3110 is 95% bamboo and 5% synthetic polymers and the absorbent layer 3120 is 75% bamboo and 25% synthetic polymers. In this embodiment, the lower liner 3130 is substantially 100% synthetic polymers. In an alternative embodiment, the upper liner 3110 is 95% bamboo and 5% a mixture of synthetic polymers and wood fibres, and the absorbent layer 3120 is 75% bamboo and 25% a mixture of synthetic polymers and wood fibres. In this alternative embodiment, the lower liner is substantially a mixture of synthetic polymers and wood fibres.

In a further alternative embodiment of the example illustrated in Figure 8, the upper liner 3110 is substantially 100% bamboo, the absorbent layer 3120 is substantially 100% bamboo and the lower liner 3130 is substantially 100% bamboo.

The invention is not limited to the specific examples or structures illustrated, a greater number of components that are illustrated in the Figures could be used, for example. For example, the thickness of the hygiene pad 100 may be from 0.1mm to 5cm. For example, the absorption rate of the hygiene pad 100 may be 0.5 seconds to 10 seconds, preferably the absorption rate may be 5 seconds to 10 seconds, more preferably the absorption rate may be 7 seconds to 8 seconds. For example, the water absorption ratio of the hygiene pad 100 may be 5 to 100, preferably the water absorption ratio may be 8 to 13, more preferably the water absorption ratio may be 9.5 to 12.1. For example, the individual bamboo fibres of the absorbent layer 120 may have a fibre length of 0.5mm to 10mm, preferably a length of 1mm to 4mm. For example, the individual bamboo fibres of the absorbent layer 120 may have an individual fibre diameter of 5µm to 50µm, preferably a diameter of 10µm to 30µm. For example, the bamboo fibre composition of the upper liner 110 or the lower liner 130 may have a fibre length of 10mm to 50mm, preferably a length of 20mm to 40mm. For example, the bamboo fibre composition of the upper liner 110 or the lower liner 130 may have a fibre diameter of 1µm to 50µm, preferably a diameter of 1µm to 30µm. For example, the upper liner may be at least 80% bamboo, at least 85% bamboo, at least 90% bamboo, or at least 95% bamboo. For example, the upper liner may be 80% to 95% bamboo. For example, the upper liner may be 90% to 95% bamboo. For example, the absorbent layer may be at least 80% bamboo, at least 85% bamboo, at least 90% bamboo, or at least 95% bamboo. For example, the absorbent layer may be 80% to 95% bamboo. For example, the absorbent layer may be 90% to 95% bamboo. For example, the lower liner may be at least 40% bamboo, at least 50% bamboo, at least 60% bamboo, at least 70% bamboo, at least 80% bamboo, at least 90% bamboo, or at least 95% bamboo. For example, the lower liner may be 80% to 95% bamboo. For example, the lower liner may be 90% to 95% bamboo. For example, the upper liner may comprise wood fibres. For example, the lower liner may comprise wood fibres. For example, the absorbent layer may comprise wood fibres. For example, the upper liner may comprise cotton fibres. For example, the lower liner may comprise cotton fibres. For example, the absorbent layer may comprise cotton fibres. For example, the upper liner may comprise synthetic polymers. For example, the lower liner may comprise synthetic polymers. For example, the absorbent layer may comprise synthetic polymers. For example, the hygiene pad may be substantially absent of wood fibres. For example, the hygiene pad may be substantially absent of cotton fibres. For example, the hygiene pad may be substantially absent of synthetic polymers. For example, the material weight of the upper liner may be 0.01g to 1g, the material weight of the absorbent layer may be 1g to 8g, and the material weight of the lower liner may be 0.01g to 1g.

## Claims

1. A biodegradable hygiene pad comprising:
an upper liner;
an absorbent layer;
a lower liner; and
a back sheet,
wherein the upper liner is arranged as a body side layer,
wherein the absorbent layer is arranged between the upper liner and the lower liner,
wherein the back sheet is arranged adjacent to the lower liner on a side opposite to the absorbent layer,
wherein the back sheet is arranged as a garment side layer,
wherein the composition of the absorbent layer is at least 75% bamboo, and
wherein the composition of the upper liner is at least 75% bamboo.

2. The biodegradable hygiene pad of claim 1, wherein the composition of the absorbent layer is at least 85% bamboo, preferably wherein the composition of the absorbent layer is at least 95% bamboo, more preferably wherein the composition of the absorbent layer is substantially 100% bamboo.

3. The biodegradable hygiene pad of claim 1 or claim 2, wherein the composition of the upper liner is at least 85% bamboo, preferably wherein the composition of the upper liner is at least 95% bamboo, more preferably wherein the composition of the upper liner is substantially 100% bamboo.

4. The biodegradable hygiene pad of any preceding claim, wherein the lower liner comprises bamboo, and/or
wherein the lower liner comprises one or more materials selected from a group of biodegradable materials comprising: wood fibres, cotton fibres, synthetic polymers and any other biodegradable material.

5. The biodegradable hygiene pad of any preceding claim further comprising a lining paper, wherein the lining paper is arranged between the upper liner and the absorbent layer.

6. The biodegradable hygiene pad of any preceding claim further comprising glue, and
optionally, wherein the glue is arranged between the upper liner and the back sheet for sealing the upper liner to the back sheet, and/or
wherein the glue is arranged between the lower liner and the back sheet for sealing the lower liner to the back sheet.

7. The biodegradable hygiene pad of any preceding claim, wherein the bamboo is bamboo viscose, and/or
wherein the bamboo comprises a hydrophilic surface layer, and/or
wherein the bamboo comprises non-woven bamboo fibres, preferably wherein the non-woven bamboo fibres are spun-lace bamboo fibres.

8. The biodegradable hygiene pad of any preceding claim, wherein the absorbent layer is substantially absent of cotton fibres.

9. The biodegradable hygiene pad of any preceding claim, wherein the absorbent layer bamboo comprises individual bamboo fibres, wherein the fibres have a fibre length of preferably 0.5mm to 10mm, more preferably a length of 1mm to 4mm.

10. The biodegradable hygiene pad of any preceding claim, wherein the back sheet comprises a biodegradable plastic, preferably wherein the biodegradable plastic comprises one or more materials selected from a group of materials comprising: polylactic acid, polybutylene adipate terephthalate, polyhydroxyalkanoates, polybutylene succinate and starch; more preferably wherein the biodegradable plastic compromises polylactic acid and polybutylene adipate terephthalate,
wherein preferably the polylactic acid comprises cornstarch, and
wherein preferably the polybutylene adipate terephthalate comprises adipic acid, terephthalic acid and butanediol.

11. The biodegradable hygiene pad of any preceding claim, wherein the hygiene pad has a thickness of preferably 0.1mm to 5cm, more preferably a thickness of 0.1mm to 1cm, and/or
wherein the hygiene pad has an absorption rate of preferably 0.5 seconds to 10 seconds, more preferably an absorption rate of 7 seconds to 8 seconds, and
wherein the hygiene pad has a water absorption ratio of preferably 5 to 100, more preferably a water absorption ratio of 9.5 to 12.1.

12. The biodegradable hygiene pad of any preceding claim, further comprising a pair of tabs for securing the hygiene pad to a garment, and/or
further comprising an adhesive portion for securing the hygiene pad to a garment.

13. A wrapped hygiene pad, wherein the hygiene pad is the biodegradable hygiene pad of any preceding claim and wherein the hygiene pad is wrapped in a biodegradable wrapper made from cornstarch.

14. A biodegradable wrapper for wrapping the biodegradable hygiene pad of any of claims 1-12, wherein the wrapper is made from cornstarch.

15. A biodegradable sanitary article according to any one of the preceding claims.
